# FASCICULE DE BREVET EUROPEEN

(11) **EP 1 034 774 B1**
(45) Date de publication et mention de la délivrance du brevet: **15.05.2002**
(21) Numéro de dépôt: 99403303.3
(22) Date de dépôt: 28.12.1999
(51) Int. Cl.: A61K 7/02, A61K 7/48

(54) **Composition cosmétique de démaquillage et/ou nettoyage sous forme d'émulsion eau-dans-huile**
Abschminkmittel oder Reinigungsmittel in der Form einer Wasser-in-Öl Emulsion
Make-up remover or cleansing composition in the form of a water-in-oil emulsion

(30) Priorité: 08.02.1999 FR 9901445
(43) Date de publication de la demande: 13.09.2000
(73) Titulaire: L'OREAL, 75008 Paris (FR)
(72) Inventeur: Lorant, Raluca, 94320 Thiais (FR)
(74) Mandataire: Rasson, Catherine

(56) Documents cités:
- EP-A- 0 463 496
- EP-A- 0 516 547
- EP-A- 0 839 521
- WO-A-96/14047

## Description

La présente invention se rapporte à une composition cosmétique de démaquillage et/ou de nettoyage sous forme d'une émulsion eau-dans-huile comprenant au moins un émulsionnant siliconé, au moins une huile hydrocarbonée à chaîne ramifiée et au moins une huile démaquillante, et à son utilisation pour le démaquillage et/ou le nettoyage de la peau, des muqueuses et/ou des yeux.

Les démaquillants et nettoyants du visage classiquement utilisés à ce jour sont des compositions ayant une concentration élevée en matières grasses dont la fonction, une fois appliquées sur la peau, est de dissoudre les différents corps gras présents sur la peau et notamment ceux qui sont contenus dans les produits de maquillage, de manière à les éliminer.

Toutefois, l'utilisation de telles compositions démaquillantes à teneur élevée en corps gras provoque, au moment de leur application, un désagrément ou inconfort qui se traduit par une sensation de lourdeur sur le visage ou de voile sur les yeux. L'application de ces compositions sur les yeux peut, par ailleurs, entraîner un gonflement des paupières.

La concentration élevée en corps gras pose également des problèmes d'odeur désagréable. Il est par conséquent nécessaire de masquer cette odeur par un parfumage intense, ce qui provoque d'autres problèmes de tolérance.

En outre, en raison de leur texture "lourde", ces démaquillants manquent de fraîcheur et sont difficiles à travailler. De plus, leur rinçage n'est pas aisé. En effet, après leur application sur la peau telle que celle des paupières, un rinçage à l'aide d'un tonique ou d'eau s'avère indispensable.

Aussi, il subsiste le besoin d'une composition nettoyante et/ou démaquillante qui ne nécessite pas de rinçage après utilisation sur la peau, qui présente une faible concentration en corps gras, évitant ainsi l'effet de "lourdeur" sur la peau, et qui nettoie et/ou démaquille la peau sans la nécessité de nombreux passages sur la peau, d'un coton imprégné de la composition.

Ainsi, la demanderesse a découvert de façon surprenante qu'une composition sous forme d'une émulsion eau-dans-huile, c'est-à-dire comprenant une phase aqueuse dispersée dans une phase huileuse, comprenant un émulsionnant siliconé, une huile hydrocarbonée à chaîne ramifiée et une huile démaquillante, permettait d'obtenir une composition fraîche et légère à l'application tout en étant efficace notamment pour nettoyer et/ou démaquiller la peau les yeux ou les muqueuses, sans aucune gêne ou irritation et avec une excellente efficacité.

Aussi, la présente invention a pour objet une composition cosmétique de démaquillage et/ou de nettoyage sous forme d'une émulsion eau-dans-huile comprenant une phase aqueuse dispersée dans une phase huileuse, caractérisée en ce qu'elle contient (1) au moins un émulsionnant siliconé, (2) au moins une huile hydrocarbonée à chaîne ramifiée et (3) au moins une huile démaquillante choisie parmi les esters d'acide gras comportant au moins 12 atomes de carbone, tels que listés dans la revendication 1.

La composition selon la présente invention présente l'avantage d'être légère et fraîche à l'application tout en réalisant un démaquillage ou un nettoyage sans rinçage obligatoire, ce qui est particulièrement intéressant pour des peaux ayant certaines affections cutanées ou dans les cas de conditions peu propices à un rinçage de la peau, tels que les voyages. De plus, elle laisse la peau douce, mate et non collante.

La composition selon l'invention permet également son emploi dans des pays chauds, où l'utilisation de démaquillants trop riches en corps gras donne une sensation de lourdeur sur la peau, souvent désagréable à supporter.

Comme émulsionnants siliconés pouvant entrer dans la composition selon l'invention, on peut citer les dimethicone copolyols et les alkyl diméthicone copolyols. Comme dimethicone copolyol, on peut citer par exemple le mélange de dimethicone copolyol et de dimethicone (polydiméthylsiloxane) (10/90) commercialisé par la société Dow Corning sous la dénomination DC3225C. Selon un mode préféré de réalisation de l'invention, on utilise comme émulsionnant siliconé un alkyl diméthicone copolyol ayant un radical alkyle comportant de 10 à 22 atomes de carbone, tel que le cétyl diméthicone copolyol comme le produit commercialisé sous la dénomination Abil EM-90 par la société Goldschmidt ; le lauryl diméthicone copolyol et par exemple le mélange d'environ 91 % de lauryl diméthicone copolyol et d'environ 9 % d'alcool isostéarylique, commercialisé sous la dénomination Q2-5200 par la société Dow Corning ; et leurs mélanges.

L'émulsionnant siliconé est de préférence utilisé en une quantité en matière active allant par exemple de 0,2 à 10 % et de préférence de 1 à 6 % en poids par rapport au poids total de la composition.

La phase huileuse de la composition selon l'invention contient au moins une huile hydrocarbonée à chaîne ramifiée et au moins une huile démaquillante choisie parmi les esters d'acide gras comportant au moins 12 atomes de carbone.

L'huile hydrocarbonée à chaîne ramifiée comporte de préférence de 10 à 20 atomes de carbone et peut être choisie par exemple dans le groupe comprenant l'isohexadécane, l'isododécane, les isoparaffines et leurs mélanges.

La quantité d'huile(s) hydrocarbonée(s) peut aller par exemple de 1 à 35 % et de préférence de 5 à 20 % en poids par rapport au poids total de la composition.

L'huile démaquillante est choisie parmi les esters d'acide gras comportant au moins 12 atomes de carbone. Ces esters sont de préférence obtenus à partir d'un alcool à chaîne droite ou ramifiée, comportant de 1 à 17 atomes de carbone et d'un acide gras à chaîne droite ou ramifiée, comportant au moins 12 atomes de carbone et de préférence de 14 à 22 atomes de carbone. Il s'agit de préférence de mono- ou di-esters.

De manière préférée, l'huile démaquillante est choisie dans le groupe des esters qui ne comportent aucune insaturation et/ou aucun groupement éther ou hydroxyle. De façon encore plus avantageuse, l'huile démaquillante est un ester saturé qui ne renferme aucun groupement éther ni hydroxyle.

Ainsi, l'huile démaquillante de la composition conforme à l'invention peut être notamment choisie dans le groupe comprenant le palmitate d'éthyl-2 hexyle (ou palmitate d'octyle), le myristate d'éthyl-2 hexyle (ou myristate d'octyle), le palmitate d'isopropyle, le myristate d'isopropyle, l'adipate de di-isopropyle, l'adipate de di-octyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle, et leurs mélanges.

La quantité d'huile(s) démaquillante(s) peut aller par exemple de 1 à 20 % et de préférence de 3 à 15 % en poids par rapport au poids total de la composition.

En outre, selon un mode de réalisation préféré de l'invention, la phase huileuse contient au moins une huile de silicone volatile qui peut être choisie par exemple parmi les cyclodiméthylsiloxanes telles que le cyclohexadiméthylsiloxane (ou cyclohexamethicone) et le cyclopentadiméthylsiloxane (ou cyclopentamethicone) et leurs mélanges.

La quantité d'huile(s) de silicone volatile(s) peut aller par exemple de 1 à 30 % et de préférence de 5 à 20 % en poids par rapport au poids total de la composition.

La phase huileuse peut contenir en outre tous les corps gras et notamment les huiles non démaquillantes autres que celles indiquées ci-dessus, classiquement utilisés dans les domaines cosmétique ou dermatologique. Comme autres huiles susceptibles d'être présentes dans la phase huileuse, on peut citer par exemple les huiles d'origine végétale comme l'huile de noyaux d'abricot, les huiles de synthèse comme le poly-isobutène hydrogéné, les huiles de silicone non volatiles et les huiles fluorées. Les autres corps gras susceptibles d'être présents dans la phase huileuse peuvent être par exemple les acides gras, les alcools gras et les cires comme la cire d'abeille.

La phase huileuse de l'émulsion peut représenter de 10 à 40 % en poids et mieux de 18 à 30 % en poids du poids total de la composition.

Selon un mode particulier de réalisation de l'invention, la composition comprend en outre un ou plusieurs esters alkylés de polyol. Comme ester alkylé de polyol utilisable dans la composition de l'invention, on peut citer notamment les esters de glycérol et/ou de sorbitan et par exemple l'isostéarate de polyglycérol, tel que le produit commercialisé sous la dénomination Isolan GI 34 par la société Goldschmidt, l'isostéarate de sorbitan, tel que le produit commercialisé sous la dénomination Arlacel 987 par la société ICI, l'isostéarate de sorbitan et de glycérol, tel que le produit commercialisé sous la dénomination Arlacel 986 par la société ICI, et leurs mélanges.

Lorsque la composition contient un ou des esters alkylés de polyol, la quantité d'ester(s) alkylé(s) de polyol peut aller par exemple de 0,05 à 5 % et mieux de 0,5 à 2 % en poids par rapport au poids total de la composition.

La composition de l'invention peut éventuellement contenir une ou plusieurs charges. La ou les charges peuvent être choisies par exemple dans le groupe formé par les particules de polyamide et notamment celles vendues sous la dénomination Orgasol par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de Polytrap ; les poudres expansées telles que les microsphères creuses et notamment, les microsphères commercialisées sous la dénomination commerciale Expancel par la société Kemanord Plast ou sous la dénomination commerciale Micropearl F 80 ED par la société Matsumoto ; les poudres de matériaux organiques naturels tels que les amidons de maïs, de blé ou de riz, réticulés ou non, telles que les poudres d'amidon réticulé par l'anhydride octénylsuccinique, commercialisées sous la dénomination Dry-Flo par la société National Starch ; les microbilles de résine de silicone telles que celles commercialisées sous la dénomination Tospearl par la société Toshiba Silicone ; et leurs mélanges.

De manière préférée, la charge est choisie parmi les microsphères commercialisées sous la dénomination commerciale Expancel, qui sont des particules de terpolymère expansé de chlorure de vinylidène, d'acrylonitrile et de méthacrylate, et notamment celles vendues sous les références 551 DE 50 (granulométrie d'environ 40 µm), 551 DE 20 (granulométrie d'environ 30 µm et masse volumique d'environ 65 kg/m³), 551 DE 12 (granulométrie d'environ 12 µm), 551 DE 80 (granulométrie d'environ 80 µm), 461 DE 50 (granulométrie d'environ 50 µm). On peut aussi utiliser des microsphères formées du même terpolymère expansé ayant une granulométrie d'environ 18 µm et une masse volumique d'environ 70 kg/m³, appelées ci-dessous EL 23. On peut aussi utiliser un mélange de ces différentes particules.

Les particules de terpolymère indiquées ci-dessus peuvent être sèches ou hydratées et peuvent être obtenues, par exemple, selon les procédés des brevets et demandes de brevets EP-A-056219, EP-A-348372, EP-A-486080, EP-A-320473, EP-A-112807 et US-A-3,615,972.

Lorsque la composition contient des charges, la quantité de charge(s) dans la composition selon l'invention peut aller de préférence de 0,01 % à 15 % et mieux de 0,1 à 5 % en poids par rapport au poids total de la composition.

La composition selon l'invention peut en outre contenir un ou plusieurs sels, et notamment un sel de magnésium tel que le sulfate de magnésium. La quantité de sel(s) peut aller par exemple de 0,1 à 5 % et de préférence de 0,5 à 1 % en poids par rapport au poids total de la composition.

En outre, de façon connue, les compositions de l'invention peuvent contenir des adjuvants habituels dans le domaine cosmétique ou dermatologique, tels que des actifs hydrophiles ou lipophiles, des conservateurs, des antioxydants, des parfums, des solvants, des charges, des filtres, des matières colorantes (pigments ou colorants), des agents basiques (triéthanolamine) ou acides et encore des vésicules lipidiques. Ces adjuvants sont utilisés dans les proportions habituelles dans le domaine cosmétique ou dermatologique, et par exemple de 0,01 à 30 % du poids total de la composition, et ils sont, selon leur nature, introduits dans la phase aqueuse ou dans la phase huileuse de l'émulsion, ou encore dans des vésicules. Ces adjuvants ainsi que leurs concentrations doivent être tels qu'ils ne modifient pas la propriété recherchée pour la composition.

Selon un mode particulier de réalisation de l'invention, la composition est de préférence fluide, c'est-à-dire qu'elle a une viscosité allant d'environ 0,2 à 3 Pa.s (2 à 30 poises) et de préférence de 0,6 à 2 Pa.s (6 à 20 poises), cette viscosité étant mesurée à environ 25°C à l'aide du viscosimètre "Rhéomat Metler" équipé d'un mobile 2 (pour les viscosités inférieures à 7 poises) ou d'un mobile 3 (pour les viscosités supérieures à 7 poises).

Toutefois, si l'on souhaite obtenir une composition moins fluide, on peut y ajouter un ou plusieurs gélifiants lipophiles, tels que les argiles modifiées comme les bentones ; les sels métalliques d'acide gras comme le stéarate d'aluminium ; la silice hydrophobe ; les esters de glycol stéarate tels que l'ester acétylé de glycol stéarate vendu sous la dénomination d'Unitwix par la société Guardian. Ces gélifiants peuvent être utilisés à des concentrations allant de 0,1 à 10 %, de préférence de 0,1 à 5 % et mieux de 0,1 à 3 % du poids total de la composition.

La composition de démaquillage et/ou de nettoyage selon l'invention contient avantageusement un milieu physiologiquement acceptable, c'est-à-dire compatible avec la peau y compris le cuir chevelu, les muqueuses (lèvres) et/ou les yeux.

La composition selon l'invention a aussi l'avantage d'avoir une excellente efficacité démaquillante du fait de la phase huileuse continue tout en étant légère, fraîche et facile à étaler, et de permettre un bon démaquillage et/ou nettoyage de la peau sans l'agresser, cette composition étant particulièrement bien adaptée aux nettoyage des peaux sèches et sensibles.

Aussi l'invention se rapporte encore à l'utilisation cosmétique de la composition telle que définie ci-dessus pour le démaquillage et/ou le nettoyage de la peau, des lèvres et/ou des yeux.

L'invention se rapporte également à un procédé cosmétique de démaquillage et/ou de nettoyage de la peau, des lèvres et/ou des yeux, caractérisé en ce que l'on applique sur la peau, les lèvres et/ou les yeux, une composition telle que décrite précédemment.

Ce procédé peut comprendre une étape de rinçage non obligatoire.

D'autres caractéristiques, aspects et avantages de la composition selon l'invention apparaîtront dans les exemples qui suivent, qui sont donnés à titre purement illustratif et nullement limitatif. Les proportions pondérales sont données en pourcentage en poids par rapport au poids total de la composition.

### Exemple 1 : Lait démaquillant pour tous types de peaux (ne tombe pas dans la portée des revendications)

### Phase huileuse :

- Cétyl dimethicone copolyol (ABIL EM 90) 2 %
- Isohexadécane 5 %
- Cyclobexamethicone 10 %
- Palmitate d'octyle 10 %

### Charge :

- Expancel 551 0,5 %

### Phase aqueuse :

- Glycérine 5 %
- Sulfate de magnésium 0,5 %
- Conservateurs 0,4 %
- Eau qsp 100 %

Mode opératoire : on disperse à l'aide d'une spatule la charge dans la phase huileuse, puis on disperse très doucement sous agitation vigoureuse la phase aqueuse dans le mélange obtenu.

On obtient ainsi un lait particulièrement agréable à utiliser et présentant de très bonnes propriétés démaquillantes. Très onctueux à l'application, il élimine en douceur le maquillage et les impuretés sans agresser ni irriter la peau qui reste douce, mate et fraîche.

### Exemple 2 : Lait démaquillant pour peaux sèches

### Phase huileuse :

- Cétyl dimethicone copolyol (ABIL EM 90) 1,5 %
- Isohexadécane 5 %
- Cyclopentamethicone 8 %
- Adipate de di-octyle 7 %
- Poly-isobutène hydrogéné 7 %

### Phase aqueuse :

- Glycérine 5 %
- Sulfate de magnésium 0,5 %
- Conservateurs 0,4 %
- Eau qsp 100 %

Mode opératoire : On prépare séparément les phases aqueuse et huileuse puis on disperse très doucement sous agitation vigoureuse la phase aqueuse dans la phase huileuse.

On obtient ainsi un lait onctueux et doux présentant de très bonnes propriétés sensorielles et démaquillantes.

### Exemple 3 : Emulsion démaquillante pour peaux sèches et sensibles

### Phase huileuse :

- Cétyl dimethicone copolyol (ABIL EM 90) 1,5 %
- Isolan GI 34 0,5 %
- Isohexadécane 5 %
- Cyclohexamethicone 5 %
- Palmitate d'isopropyle 5 %
- Huile d'abricot 5 %
- Cire d'abeille 1 %

### Phase aqueuse :

- Glycérine 5 %
- Sulfate de magnésium 0,5 %
- Conservateurs 0,4 %
- Eau qsp 100 %

Mode opératoire : On prépare séparément à chaud les phases aqueuse et huileuse puis on disperse très doucement sous agitation vigoureuse la phase aqueuse dans la phase huileuse.

On obtient une crème souple, fondante sur la peau. Douce à l'application, elle s'étale facilement et permet l'élimination du maquillage et des impuretés en douceur et sans frotter, par simple essuyage avec un coton. La peau reste nette, veloutée et est apaisée.

## Revendications

1. Composition cosmétique de démaquillage et/ou de nettoyage sous forme d'une émulsion eau-dans-huile comprenant une phase aqueuse dispersée dans une phase huileuse, **caractérisée en ce qu'**elle contient (1) au moins un émulsionnant siliconé choisi parmi les alkyl diméthicone copolyols ayant un radical alkyle comportant de 10 à 22 atomes de carbone, (2) au moins une huile hydrocarbonée à chaîne ramifiée et (3) au moins une huile démaquillante choisie parmi le palmitate d'éthyl-2 hexyle, le myristate d'éthyl-2 hexyle, le palmitate d'isopropyle, le myristate d'isopropyle, l'adipate de di-isopropyle, l'adipate de di-octyle, l'hexanoate d'éthyl-2 hexyle, le laurate d'éthyle, le myristate de méthyle, l'octanoate d'octyldodécyle, le néopentanoate d'isodécyle, le myristate d'éthyle, le propionate de myristyle, l'éthyl-2 hexanoate d'éthyl-2 hexyle, l'octanoate d'éthyl-2 hexyle, le caprate/caprylate d'éthyl-2 hexyle, le palmitate de méthyle, le myristate de butyle, le myristate d'isobutyle, le palmitate d'éthyle, le laurate d'isohexyle, le laurate d'hexyle, l'isostéarate d'isopropyle et leurs mélanges.

2. Composition selon la revendication 1, **caractérisée en ce que** l'émulsionnant siliconé est choisi parmi le cétyl diméthicone copolyol, le lauryl diméthicone copolyol et leurs mélanges.

3. Composition selon l'une quelconque des revendications précédentes, **caractérisée en ce que** l'émulsionnant siliconé représente de 0,2 à 10 % en poids en matière active, par rapport au poids total de la composition.

4. Composition selon l'une quelconque des revendications précédentes, **caractérisée en ce que** l'huile hydrocarbonée à chaîne ramifiée est choisie dans le groupe comprenant l'isohexadécane, l'isododécane, les isoparaffines et leurs mélanges.

5. Composition selon l'une quelconque des revendications précédentes, **caractérisée en ce que** la quantité d'huile(s) hydrocarbonée(s) va de 1 à 35 % en poids par rapport au poids total de la composition.

6. Composition selon l'une quelconque des revendications précédentes, **caractérisée en ce que** la quantité d'huile(s) démaquillante(s) va de 1 à 20 % en poids par rapport au poids total de la composition.

7. Composition selon l'une quelconque des revendications précédentes, **caractérisée en ce qu'**elle contient en outre au moins une huile de silicone volatile.

8. Composition selon la revendication 7, **caractérisée en ce que** la quantité d'huile(s) de silicone volatile(s) va de 1 à 30 % en poids par rapport au poids total de la composition.

9. Composition selon l'une quelconque des revendications précédentes, **caractérisée en ce que** la phase huileuse représente de 10 à 40 % en poids du poids total de la composition.

10. Composition selon l'une quelconque des revendications précédentes, **caractérisée en ce qu'**elle contient en outre un ester alkylé de polyol.

11. Composition selon la revendication précédente, **caractérisée en ce que** la quantité d'ester(s) alkylé(s) de polyol va de 0,05 à 5 % en poids par rapport au poids total de la composition.

12. Composition selon l'une quelconque des revendications précédentes, **caractérisée en ce qu'**elle contient en outre au moins une charge.

13. Composition selon la revendication précédente, **caractérisée en ce que** la quantité de charge(s) va de 0,01 % à 15 % en poids par rapport au poids total de la composition.

14. Utilisation cosmétique de la composition selon l'une quelconque des revendications précédentes pour le démaquillage et/ou le nettoyage de la peau, des lèvres et/ou des yeux.

15. Procédé cosmétique de démaquillage et/ou de nettoyage de la peau, des lèvres et/ou des yeux, **caractérisé en ce que** l'on applique sur la peau, les lèvres et/ou les yeux, une composition selon l'une quelconque des revendications 1 à 13.

## Claims

1. Make-up removing and/or cleansing cosmetic composition in the form of a water-in-oil emulsion comprising an aqueous phase dispersed in an oily phase, **characterized in that** it comprises (1) at least one silicone emulsifier chosen from alkyl dimethicone copolyols having an alkyl radical comprising from 10 to 22 carbon atoms, (2) at least one branched-chain hydrocarbonaceous oil and (3) at least one make-up removing oil chosen from 2-ethylhexyl palmitate, 2-ethylhexyl myristate, isopropyl palmitate, isopropyl myristate, diisopropyl adipate, dioctyl adipate, 2-ethylhexyl hexanoate, ethyl laurate, methyl myristate, octyldodecyl octanoate, isodecyl neopentanoate, ethyl myristate, myristyl propionate, 2-ethylhexyl 2-ethylhexanoate, 2-ethylhexyl octanoate, 2-ethylhexyl caprate/caprylate, methyl palmitate, butyl myristate, isobutyl myristate, ethyl palmitate, isohexyl laurate, hexyl laurate, isopropyl isostearate and their mixtures.

2. Composition according to Claim 1, **characterized in that** the silicone emulsifier is chosen from cetyl dimethicone copolyol, lauryl dimethicone copolyol and their mixtures.

3. Composition according to either one of the preceding claims, **characterized in that** the silicone emulsifier represents from 0.2 to 10% by weight as active material with respect to the total weight of the composition.

4. Composition according to any one of the preceding claims, **characterized in that** the branched-chain hydrocarbonaceous oil is chosen from the group consisting of isohexadecane, isododecane, isoparaffins and their mixtures.

5. Composition according to any one of the preceding claims, **characterized in that** the amount of hydrocarbonaceous oil(s) ranges from 1 to 35% by weight with respect to the total weight of the composition.

6. Composition according to any one of the preceding claims, **characterized in that** the amount of make-up removing oil(s) ranges from 1 to 20% by weight with respect to the total weight of the composition.

7. Composition according to any one of the preceding claims, **characterized in that** it additionally comprises at least one volatile silicone oil.

8. Composition according to Claim 7, **characterized in that** the amount of volatile silicone oil(s) ranges from 1 to 30% by weight with respect to the total weight of the composition.

9. Composition according to any one of the preceding claims, **characterized in that** the oily phase represents from 10 to 40% by weight of the total weight of the composition.

10. Composition according to any one of the preceding claims, **characterized in that** it additionally comprises a polyol alkyl ester.

11. Composition according to the preceding claim, **characterized in that** the amount of polyol alkyl ester(s) ranges from 0.05 to 5% by weight with respect to the total weight of the composition.

12. Composition according to any one of the preceding claims, **characterized in that** it additionally comprises at least one filler.

13. Composition according to the preceding claim, **characterized in that** the amount of filler(s) ranges from 0.01% to 15% by weight with respect to the total weight of the composition.

14. Cosmetic use of the composition according to any one of the preceding claims in removing make-up from and/or cleansing the skin, lips and/or eyes.

15. Cosmetic process for removing make-up from and/or cleansing the skin, lips and/or eyes, **characterized in that** a composition according to any one of Claims 1 to 13 is applied to the skin, lips and/or eyes.

## Patentansprüche

1. Kosmetische Zusammensetzung zum Abschinken und/oder zur Reinigung, die in Form einer Wasser-in-Öl-Emulsion vorliegt und eine in einer Ölphase dispergierte wäßrige Phase enthält, **dadurch gekennzeichnet, daß** sie (1) mindestens einen Siliconemulgator, der unter den Alkyldimethiconcopolyolen ausgewählt ist, die eine Alkylgruppe mit 10 bis 22 Kohlenstoffatomen aufweisen, (2) mindestens ein Kohlenwasserstofföl mit verzweigter Kette und (3) mindestens ein Öl zum Abschminken enthält, das unter 2-Ethylhexylpalmitat, 2-Ethylhexylmyristat, Isopropylpalmitat, Isopropylmyristat, Diisopropyladipat, Dioctyladipat, 2-Ethylhexylhexanoat, Ethyllaurat, Methylmyristat, Octyldodecyloctanoat, Isodecylneopentanoat, Ethylmyristat, Myristylpropionat, 2-Ethylhexyl-2-ethylhexanoat, 2-Ethylhexyloctanoat, 2-Ethylhexylcaprat/caprylat, Methylpalmitat, Butylmyristat, Isobutylmyristat, Ethylpalmitat, Isohexyllaurat, Hexyllaurat, Isopropylisostearat und deren Gemischen ausgewählt ist.

2. Zusammensetzung nach Anspruch 1, **dadurch gekennzeichnet, daß** der Siliconemulgator unter Cetyldimethiconcopolyol, Lauryldimethiconcopolyol und deren Gemischen ausgewählt ist.

3. Zusammensetzung nach einem der vorhergehenden Ansprüche, dadurch gekennzcichnet, daß der Siliconemulgator 0,2 bis 10 Gew.-% (Wirkstoff), bezogen auf das Gesamtgewicht der Zusammensetzung, ausmacht.

4. Zusammensetzung nach einem der vorhergehenden Ansprüche, **dadurch gekennzeichnet, daß** das Kohlenwasserstofföl mit verzweigter Kette unter Isohexadecan, Isododecan, den Isoparaffinen und deren Gemischen ausgewählt ist.

5. Zusammensetzung nach einem der vorhergehenden Ansprüche, **dadurch gekennzeichnet, daß** der Mengenanteil des Kohlenwasserstofföls oder der Kohlenwasserstofföle im Bereich von 1 bis 35 Gew.-%, bezogen auf das Gesamtgewicht der Zusammensetzung, liegt.

6. Zusammensetzung nach einem der vorhergehenden Ansprüche, **dadurch gekennzeichnet, daß** der Mengenanteil des Öls oder der Öle zum Abschminken im Bereich von 1 bis 20 Gew.-%, bezogen auf das Gesamtgewicht der Zusammensetzung, liegt.

7. Zusammensetzung nach einem der vorhergehenden Ansprüche, **dadurch gekennzeichnet, daß** sie ferner mindestens ein flüchtiges Siliconöl enthält.

8. Zusammensetzung nach Anspruch 7, **dadurch gekennzeichnet, daß** der Mengenanteil des flüchtigen Siliconöls oder der flüchtigen Siliconöle im Bereich von 1 bis 30 Gew.-%, bezogen auf das Gesamtgewicht der Zusammensetzung, liegt.

9. Zusammensetzung nach einem der vorhergehenden Ansprüche, **dadurch gekennzeichnet, daß** die Ölphase 10 bis 40 Gew.-% des Gesamtgewichts der Zusammensetzung ausmacht.

10. Zusammensetzung nach einem der vorhergebenden Ansprüche, **dadurch gekennzeichnet, daß** sie außerdem einen Polyolalkylester enthält.

11. Zusammensetzung nach dem vorhergehenden Anspruch, **dadurch gekennzeichnet, daß** der Mengenanteil des Alkylesters oder der Alkylester von Polyolen im Bereich von 0,05 bis 5 Gew.-%, bezogen auf das Gesamtgewicht der Zusammensetzung, liegt.

12. Zusammensetzung nach einem der vorhergehenden Ansprüche, **dadurch gekennzeichnet, daß** sie außerdem mindestens einen Füllstoff enthält.

13. Zusammensetzung nach dem vorhergehenden Anspruch, **dadurch gekennzeichnet, daß** der Mengenanteil des Füllstoffs oder der Füllstoffe im Bereich von 0,01 bis 15 Gew.-%, bezogen auf das Gesamtgewicht der Zusammensetzung, liegt.

14. Kosmetische Verwendung der Zusammensetzung nach einem der vorhergehenden Ansprüche zum Abschinken und/oder zur Reinigung der Haut, der Lippen und/oder der Augen.

15. Kosmetisches Verfahren zum Abschminken und/oder zur Reinigung der Haut, der Lippen und/oder der Augen, **dadurch gekennzeichnet, daß** auf die Haut, die Lippen und/oder die Augen eine Zusammensetzung nach einem der Ansprüche 1 bis 13 aufgetragen wird.
